Europäisches Patentamt

European Patent Office

Office européen des brevets

(19)

(11) **EP 1 200 187 B1**

(12) **EUROPÄISCHE PATENTSCHRIFT**

(45) Veröffentlichungstag und Bekanntmachung des Hinweises auf die Patenterteilung:
**08.12.2004 Patentblatt 2004/50**

(21) Anmeldenummer: **00947956.9**

(22) Anmeldetag: **09.07.2000**

(51) Int Cl.[7]: **B01J 21/16**, C07C 1/20, C07C 1/24

(86) Internationale Anmeldenummer:
**PCT/EP2000/006507**

(87) Internationale Veröffentlichungsnummer:
**WO 2001/008796 (08.02.2001 Gazette 2001/06)**

(54) **VERFAHREN ZUR HERSTELLUNG VON KATALYSATOREN DURCH SÄUREAKTIVIERUNG**

METHOD FOR PRODUCING CATALYSTS BY ACID ACTIVATION

PROCEDE DE PREPARATION DE CATALYSEURS PAR ACTIVATION ACIDE

(84) Benannte Vertragsstaaten:
**AT BE CH CY DE DK ES FI FR GB GR IE IT LI LU MC NL PT SE**

(30) Priorität: **30.07.1999 DE 19935914**

(43) Veröffentlichungstag der Anmeldung:
**02.05.2002 Patentblatt 2002/18**

(73) Patentinhaber: **SÜD-CHEMIE AG**
**80333 München (DE)**

(72) Erfinder: **FLESSNER, Uwe**
**D-81375 München (DE)**

(74) Vertreter: **Westendorp, Michael, Dr. et al**
**Patentanwälte**
**Splanemann Reitzner**
**Baronetzky Westendorp**
**Rumfordstrasse 7**
**80469 München (DE)**

(56) Entgegenhaltungen:
**US-A- 2 840 618          US-A- 2 985 595**
**US-A- 4 193 454**

- **DATABASE WPI Section Ch, Week 197818 Derwent Publications Ltd., London, GB; Class E19, AN 1978-32832A XP002148655 & JP 53 030996 A (CHIYODA CHEM ENG CONSTR CO), 23. März 1978 (1978-03-23)**
- **DATABASE WPI Section Ch, Week 197737 Derwent Publications Ltd., London, GB; Class E36, AN 1977-65855Y XP002148656 & JP 52 092891 A (CHIYODA CHEM ENG CONSTR CO), 4. August 1977 (1977-08-04)**
- **LASZLO: 'CATALYSIS OF FRIEDEL-CRAFTS ALKYLATION BY A MONTMORILLONITE DOPED WITH TRANSITION METAL OXIDE' HELVETICA CHIMICA ACTA Bd. 70, 1987, Seite 577**
- **CATIVIELA ET AL: 'CLAY-CATALYZED FRIEDEL-CRAFTS ALKYLATION OF ANISOLE WITH DIENES' APPL. CATAL A: GENERAL Bd. 123, 1995, Seite 273**

Bemerkungen:
Die Akte enthält technische Angaben, die nach dem Eingang der Anmeldung eingereicht wurden und die nicht in dieser Patentschrift enthalten sind.

## Beschreibung

Die Erfindung betrifft ein Verfahren zur Herstellung von Katalysatoren durch Säureaktivierung von Schichtsilicaten und Modifizierung mit katalytisch wirksamen Metallionen.

Katalysatoren auf der Basis von Schichtsilicaten, z. B. von Tonen, werden in vielen technischen Reaktionen eingesetzt. Natürlich vorkommende Tone, wie z.B. Montmorillonit, Kaolin oder Attapulgit, weisen aber zum Teil zu geringe Aktivitäten auf. Aus diesem Grund werden die Tone zur Herstellung von Katalysatoren oft durch Behandeln mit Säure aktiviert. Dabei kann auf der einen Seite die reine Belegung mit z.B. Schwefelsäure und zum anderen die Aktivierung durch eine Extraktion des Rohtons mit Säure, meist Schwefel- oder Salzsäure, erfolgen.

So wird nach der US-A-3,452,056 ein säurebelegter Montmorillonit-Katalysator (KSF/O) zur Alkylierung von Diphenylamin verwendet. Nach der US-A-5,672,752 wird für dieselbe Reaktion ein säureextrahierter Montmorillonit verwendet. Katalysatoren dieses Typs sind unter der Handelsbezeichnung Retrol®, Fulcat® und K10 erhältlich. Nach der US-A-5,043,511 werden als Ersatz von korrosiven Homogenkatalysatoren , wie z.B. $AlCl_3$ oder $BCl_3$, heterogene Katalysatoren eingesetzt, die durch Coextrusion von Tonen mit zwei verschiedenen Metallsalzen und thermischer Behandlung bei Temperaturen von 300°C bis 800°C hergestellt werden. Derart hergestellte Produkte werden als Katalysatoren für die Alkylierung von z.B. Benzol mit Olefinen verwendet.

Aber auch diese modifizierten Tone weisen Nachteile auf, wie z.B. eine rasche Deaktivierung oder ein aufwendiges Herstellungsverfahren, und es fehlte nicht an Bemühungen, diese zu eliminieren. So berichtet die US-A-2,464,127 von einem zweistufigen Verfahren mit dem Ziel, im Endprodukt der Säureaktivierung von Montmorillonit möglichst geringe Mengen Eisen zu erhalten. Nach der US-A-2,574,895 wird ein Teil der bei der Säurebehandlung extrahierten Salze wieder auf das montmorillonithaltige Material aufgefüllt, wobei aber reduzierende Reagentien verwendet werden, die verhindern sollen, daß im Fällprozeß Eisen niedergeschlagen wird. Beiden Anmeldungen gemein ist das Bemühen, die Standzeit der resultierenden Katalysatoren durch eine verminderte Koksbildung zu erhöhen.

Die DE-A-1 271 682 beschreibt ein Verfahren zur Aktivierung von Montmorilloniten durch Säureextraktion in Anwesenheit von inerten organischen flüssigen Verbindungen. Zur Säureaktivierung werden starke Säuren, wie z.B. Salz- oder Schwefelsäure verwendet. Durch die Anwesenheit der organischen Bestandteile während des Säureaufschlusses des Tones werden die Netzebenenabstände des resultierenden Montmorillonit-Katalysators vergrößert, wodurch mehr Katalysezentren für das Substrat zugänglich werden. Die Katalysatoren nach der DE-A-1 271 682 werden zur Alkylierung von phenolischen Verbindungen eingesetzt.

Andere Wege der Aktivierung beschreibt die EP-A-352 878. Hierbei wird ein unbehandelter Ton durch Imprägnieren mit z.B. Zink-, Kupfer- oder Nickelsalzen belegt. Dabei werden organische Lösungen der Metallsalze verwendet; das Lösungsmittel wird nach der Imprägnierung destillativ entfernt. Nach der EP-A-144 219 und der EP-A-031 252 werden Rohtone durch eine ähnliche Imprägnierungstechnik bzw. durch einen Ionenaustausch aktiviert. Beim Ionenaustausch werden die natürlichen Interkalat-Kationen der Mineralien, hauptsächlich Natrium, Calcium und Magnesium, gegen katalytisch aktive Metalle substituiert. Nachteilig bei diesen Verfahren ist jedoch, daß die derart hergestellten Katalysatoren ein zu geringes Porenvolumen besitzen. Dies bedeutet somit, daß den Substraten nur die äußere Oberfläche zugänglich ist. Die überwiegenden inneren Oberflächen der Katalysatoren sind aber nicht zugänglich, wodurch ein großer Teil an möglicher Aktivität ungenutzt bleibt.

Um diesen Nachteil zu überwinden, beschreibt Laszlo in Helvetica Chimica Acta 70 (1987) 577 die nachträgliche Metallionenaktivierung von bereits säureaktivierten Montmorilloniten. So wird der kommerziell erhältliche, porenreiche Katalysator K10, der durch Säureaktivierung von Bentonit hergestellt wird, durch Ionenaustausch mit verschiedenen Metallsalzen modifiziert. Dazu wird das als Pulver in einer methanolischen Slurry vorliegende K10 mit Lösungen der Metallsalze für mehrere Stunden behandelt. Die Menge der verwendeten Metallionen ist so berechnet, daß diese in etwa 30- bis 40-fachem Überschuß, bezogen auf die Ionenumtauschfähigkeit des K10, vorliegen. Dann wird das Gemisch durch Filtration getrennt und der Katalysator salzfrei gewaschen und anschließend getrocknet. Besonders gute Effekte werden durch einen Ionenaustausch mit Aluminium- und Titan-Salzen erreicht, ein Austausch mit Eisensalzen führt nur in wenigen Fällen zu einer verbesserten Reaktivität des Katalysators.

Ein ähnliches Verfahren wird auch von Cativiela in Appl. Cat. A 123 (1995) 273 angewendet. Cativiela calciniert die Katalysatoren noch zusätzlich bei Temperaturen um 550°C, um die Brönsted-Aktivität herabzusetzen. In dieser Durckschrift werden besonders mit Cer-Salzen gute Aktivitäten erzielt. Der Austausch mit Eisenionen zeigt jedoch keine besonderen Effekte.

Das bei diesen Aktivierungsmethoden angewendete Verfahren hat wenigstens zwei Nachteile. Zum einen besteht der Prozeß aus zwei voneinander unabhängigen Teilprozessen, nämlich die Säureaktivierung von Rohtonen und die anschließende Ionenumtauschreaktion. Zum zweiten muß die Ionenumtauschreaktion bei hohem Ionenüberschuß durchgeführt werden, was zwangsläufig zu hochbelasteten Abwasserströmen führt. Zusätzlich sind die für die Reinigung des Vorprodukts nach der Säureaktivierung und für das Waschen nach dem Ionenaustausch benötigten Waschwassermengen, sehr hoch, so daß dadurch große Mengen Abwasser anfallen.

Die EP-B 284 397 beschreibt ein Verfahren, bei dem der durch Ionenaustausch zu aktivierende Ton in einem vorgeschalteten Schritt mit Lithiumionen ausgetauscht und anschließend thermisch behandelt wird. Mit dem entstehenden Zwischenprodukt wird dann in einem zweiten Verfahrensschritt ein Ionenumtausch durchgeführt. Bevorzugt verwendete Metallionen dafür sind Aluminiumionen; Li-Tone mit eingetauschten Eisenionen zeigen keine verbesserte Aktivität im Vergleich zum Ausgangsmaterial.

Die US-A-4,196,102 beschreibt die Herstellung eines Hydrierkatalysators auf Sepiolithbasis. Dazu wird Sepiolith mit hydrieraktiven Metallsalzen, wie Kobalt, Nickel, Eisen oder Kupfer imprägniert und nach einem Waschschritt bei Temperaturen von <1000 °C calciniert. Der Sepiolith wird dabei keiner Säureaktivierung unterzogen.

Die US 2,840,618 betrifft ein Verfahren zur Herstellung eines Katalysators zur Verwendung bei der Herstellung von Alkinolen durch Umsetzung einer Carbonylverbindung mit Acetylenkohlenwasserstoffen. Nach diesem Verfahren wird Kupferoxid auf einem kieselsäurehaltigen Träger abgeschieden. Der so imprägnierte kieselsäurehaltige Träger wird auf eine Temperatur im Bereich von 400 bis 800 °C erhitzt, bis sich in der Oberflächenschicht Kupfersilicat gebildet hat. Der hitzebehandelte Katalysator bildet kein rotes Kupferacetylid, wenn er bei Raumtemperatur mit Acetylen behandelt wird. Der kieselsäurehaltige Träger wird dadurch hergestellt, dass ein Gemisch aus Kaolin und einem wasserlöslichen organischen Bindemittel zu einem Formkörper geformt wird und der Formkörper in Gegenwart von Luft bei Temperaturen von 800 bis 850 °C erhitzt wird, um das Bindemittel auszubrennen und um den Kaolin in Montmorillonit umzuwandeln, worauf der Formkörper auf Temperaturen von etwa 1000 bis 1200 °C erhitzt wird, um einen porösen kieselsäurehaltigen Träger zu erhalten, der das Röntgenbeugungsmuster des Mullits aufweist. Von einer Säureaktivierung des Trägers in Anwesenheit von Metallsalzen ist nicht die Rede. Außerdem wird bei Temperaturen von mehr als 1000 °C das Schichtsilicatgitter zerstört. Der erhaltene Mullit ist kein Schichtsilicat mehr.

In der US 4,193,454 beschreibt ein Verfahren zur Herstellung eines hoch aktiven Katalysators, wobei ein unbehandeltes Tonmaterial mit einer verdünnten Metallsalzlösung behandelt wird. Das für die Herstellung des Katalysators verwendete Tonmaterial soll vor der Beladung mit Metallionen noch keinen Temperaturen von mehr als 100 °C ausgesetzt worden sein. Nach erfolgtem Ionenaustausch wird das Tonmaterial auf eine Temperatur von mindestens 50 °C und höchstens 250 °C erhitzt. Auch bei diesem Verfahren findet keine Säureaktivierung des Tonmaterials statt.

Die US 4,590,294 betrifft ein Verfahren zur Herstellung von Estern aus ungesättigten Kohlenwasserstoffen und Carbonsäuren. Die Reaktion wird in Gegenwart eines Schichtsilicats durchgeführt, das aus der Gruppe von Bentonit und Montmorillonit ausgewählt ist. Die Schichtsilicate werden durch Protonenaustausch aktiviert. Der Ionenaustausch wird unter milden Bedingungen durchgeführt, sodass die Schichtstruktur der Silikate nicht angegriffen wird. Auch bei diesem Verfahren wird ein Schichtsilicat eingesetzt, das keiner Säureaktivierung unterzogen wurde.

Die EP 0 925 829 A1 beschreibt einen aktivierten Ton zur Behandlung aromatischer Kohlenwasserstoffe, der durch Behandlung von dioktaedrischen Smektit-Tonmineralien mit einer Säure erhalten wurde, der eine spezifische BET-Oberfläche von nicht mehr als 250 m$^2$/g hat und eine Eisenkomponente in einer Menge von nicht kleiner als 2,5 Gew.-%, berechnet als $Fe_2O_3$, enthält.

Die US 4,774,213 beschreibt die Herstellung eines eisenhaltigen Katalysators zur Erniedrigung des NO-Gehaltes in Abgasen. Diese eisenhaltigen Katalysatoren werden auf Basis säureaktivierter Tone hergestellt, wobei jedoch ein Austausch mit Ionen nur in Kombination mit der nachfolgenden Hydrolyse erfolgt. Weiterhin werden die dadurch entstehenden Zwischenprodukte durch Calcinierung bei hohen Temperaturen oxidiert, sodass das ausgetauschte, gefällte Eisen als $Fe_2O_3$ und nicht in Form von $Fe^{3+}$-Ionen vorliegt.

In der WO 94/29020 wird die klassische Aktivierung von Schichtsilicaten mit Säuren sowie eine entsprechende Umformung zu kugelförmigen Produkten beschrieben. Die dabei erhaltenen Katalysatoren werden für verschiedene Reaktionen verwendet, u.a. auch zur Alkylierung bzw. Aromatenbehandlung.

Die US 4,329,257 beschreibt die Herstellung von mit Phosphorsäure imprägnierten Katalysatoren zur Hydratation von C2 bis C3-Olefinen. Das Verfahren sieht eine milde Säureaktivierung von Schichtsilicaten vor, die anschließend mit Metalloxiden der 6. Nebengruppe zusammen verformt und dann bei hohen Temperaturen miteinander calciniert werden. Die dadurch entstandenen Produkte werden erneut mit Säure ausgelaugt. Weiterhin werden die Träger noch mit 35 Gew.-% Phosphorsäure belegt.

Aufgabe der vorliegenden Erfindung war es nun, mit Metallionen modifizierte Katalysatoren herzustellen, wobei einfache Verfahrensschritte und geringe Abwasserströme gewährleistet werden sollten.

Gegenstand der Erfindung ist somit ein Verfahren zur Herstellung von Katalysatoren durch Säureaktivierung von Schichtsilicaten und Modifizierung mit katalytisch wirksamen Metallionen, das dadurch gekennzeichnet ist, dass man die Säureaktivierung in Anwesenheit von zugesetzten katalytisch wirksamen Aluminium- und/oder Cerionen durchführt und die bei der Säureaktivierung entstehende Lösung zusammen mit der die überschüssigen, katalytisch wirksamen Kationen enthaltenden Restlösung abtrennt.

Bevorzugte Ausführungsformen des erfindungsgemäßen Verfahrens sind in den Unteransprüchen angegeben.

Überraschenderweise stellte sich dabei heraus, dass bei einer Säureaktivierung von Schichtsilicaten in Anwesenheit der aktivierenden Ionen hochaktive Katalysatoren erhalten werden können. Dabei ist es nicht nötig, die Aktivierung und den Ionenaustausch in getrennten Verfahrensschritten durchzuführen. Völlig unerwar tet reichen bei dem erfindungsgemäßen Verfahren bereits geringe Mengen an katalytisch wirksamen Ionen aus, wodurch die Prozesskosten, aber auch die Umweltbelastung des Verfahrens auf ein Minimum reduziert werden kann. In bestimmten Fällen, wie z. B. bei der Aktivierung mit Aluminiumionen, können die derart erhaltenen Ablaugen sogar als Fällungshilfsmittel bei der Reinigung von kommunalen Abwässern eingesetzt werden. Es stellte sich weiterhin heraus, dass die zur Modifikation verwendeten Metallionen in einem besonders hohen Aktivierungsgrad vorliegen, sodass die im Endprodukt vorliegende Metallionenmenge besonders gering gehalten werden kann.

Gegenstand der Erfindung ist auch die Verwendung der nach dem erfindungsgemäßen Verfahren erhältlichen Katalysatoren für protonenkatalysierte Reaktionen, insbesondere zur Umsetzung von höheren Olefinen mit aromatischen Hydroxyverbindungen und Aminen, für Veresterungs- und Dehydratisierungsreaktionen, sowie für die Reinigung von Xylol und für lewissäure-katalysierte Reaktionen wie z. B. die Alkylierung von Aromaten.

Die Erfindung ist durch die nachstehenden Beispiele erläutert.

Beispiel 1

Vergleichskatalysator

Ein vorgetrockneter bayerischer montmorillonithaltiger Rohton mit einer Ionenumtauschfähigkeit (IUF) von 80 mVal/100g wurde durch Salzsäurebehandlung aufgeschlossen.

Zur Bestimmung der Ionenumtauschfähigkeit (IUF) wird das zu untersuchende Schichtsilicat über einen Zeitraum von 2 Stunden bei 150°C getrocknet. Anschließend wird das getrocknete Material mit einem Überschuß an wäßriger 2N $NH_4Cl$-Lösung eine Stunde unter Rückfluß zur Reaktion gebracht. Nach einer Standzeit von 16 Stunden bei Raumtemperatur wird filitriert, worauf der Filterkuchen gewaschen, getrocknet und gemahlen und der $NH_4$-Gehalt im Schichtsilicat durch Stickstoffbestimmung (CHN-Analysator der Fa. Leco) ermittelt wird. Der Anteil und die Art der ausgetauschten Metallionen wird im Filtrat durch ICP-Spektroskopie bestimmt.

Beim Aufschluß wurden 90,3 g des Rohtones mit einem Wassergehalt von 16,9 Gew.-% zusammen mit 250,7g Wasser und 87,5g 30%-iger Salzsäure in einem Dreihalskolben mit Rückflußkühler 8h bei Siedetemperatur behandelt. Danach wurde die Mutterlauge vom Produkt durch Filtration mittels einer Filternutsche abgetrennt und unter Verwendung von entmineralisiertem Wasser solange gewaschen, bis kein Chlorid mehr im Waschwasser nachweisbar war. Der gewaschene Filterkuchen wurde bei einer Temperatur von 120°C getrocknet und anschließend vermahlen.

Das so erhaltene Produkt besaß eine BET-Oberfläche von 253 $m^2$/g (nach DIN 66131) und ein Porenvolumen von 0,403 ml/g (bestimmt durch Stickstoffadsorption und Auswertung der Adsorptionsisotherme nach der BJH-Methode - E.P. Barrett et al., J.Am.Chem.Soc.<u>73</u> (1951) 373). Die aus der BJH-Ableitung gewonnene Porenverteilungskurve zeigte eine Gauß'sche Verteilung mit einem Maximum bei 5,5 nm. Das Material enthielt unter anderen folgende austauschfähige Metallionen :

| | |
|---|---|
| $Fe^{3+}$ | 1,0 mVal/100 g |
| $Al^{3+}$ | 11,4 mVal/100 g |
| $Ce^{3+}$ | < 0,1 mVal/100 g |

Beispiel 2

Aluminiumhaltiger Katalysator

Ein bayerischer montmorillonithaltiger Rohton mit einer IUF von 92 meq/100g wurde analog zu Beispiel 1 aktiviert. Zusätzlich zu den in Beispiel 1 angegebenen Reagenzien wurde dem Ansatz 7,24 g $AlCl_3$ * 6 $H_2O$ in Form einer konzentrierten Lösung zugegeben. Für diesen Ansatz wurden neben dem Aluminiumchlorid eingesetzt:

| | |
|---|---|
| Rohton (17,2Gew.-% $H_2O$) | 82,8 g |
| Wasser | 250,3 g |
| HCl (30 %-ig) | 50,0 g |

Das derart erhaltene Produkt besaß eine BET Oberfläche von 315 $m^2$/g und ein BJH-Porenvolumen von 0,425 ml/g. Das Maximum der Porenverteilungskurve lag bei 3,3 nm. Das Produkt enthielt 18,0 meq/100 g austauschbares $Al^{3+}$.

<u>Beispiel 3</u>

<u>Cerhaltiger Katalysator</u>

Ein montmorillonithaltiger Rohton aus der Türkei wurde auf einen Wassergehalt von etwa 15 Gew.-% getrocknet und vermahlen. Das Material mit einem resultierenden Wassergehalt von 13,1 Gew.-% wurde wie in Beispiel 1 beschrieben aktiviert, wobei das Reaktionsgemisch mit $Ce(NO_3)_3 * 6 H_2O$ angereichert wurde. Für diesen Ansatz wurden eingesetzt:

| | |
|---|---|
| Rohton (13,1 Gew.-% $H_2O$) | 86,3 g |
| Wasser | 254,6 g |
| HCl (30Gew.-%ig) | 62,5 g |
| $Ce(NO_3)_3 * 6 H_2O$ | 13,03 g |

Die Analyse des Produktes ergab eine BET-Oberfläche von 379 $m^2$/g und ein BJH-Porenvolumen von 0,431 ml/g. Das Maximum der Porenverteilungskurve lag bei 3,0 nm. Das Produkt enthielt 5,1 meq/100 g austauschbares $Ce^{3+}$.

<u>Beispiel 4</u>

<u>Umsetzung von Diphenylamin mit Nonen</u>

42,5 g (0,25 mol) Diphenylamin wurde in einem 500 ml-Dreihalskolben auf etwa 150 °C aufgeheizt und aufgeschmolzen. Zu der Schmelze wurden 5,0 g Katalysator und 44,2 g (0,35 mol) Nonen zugegeben. Nach einer Reaktionszeit von 4 h wurden weitere 41,6 g (0,33 mol) Nonen zugegeben, wobei die Reaktionstemperatur von 150 °C beibehalten wurde. Nach einer Reaktionszeit von 8 h wurde das Reaktionsgemisch vom Katalysator durch Filtration abgetrennt. Die Ausbeute an dialkyliertem Diphenylamin wurde infrarotspektroskopisch unter Verwendung der Formel

$$(\%) \text{ Dialkylat} = [\text{Log(ext@ 820 cm}^{-1}/\text{ext@ 743cm}^{-1}) + 1,141]/0,019;$$

ext@ Extinktion (absorbance) bei der angegebenen Wellenzahl bestimmt. Hierbei wurde berücksichtigt, dass das Adsorptionsmaximum bei 820 $cm^{-1}$ mit den dialkylierten Produkten, das Adsorptionsmaximum bei 743 $cm^{-1}$ mit den monoalkylierten Produkten korrespondiert. Zur Bestimmung der Extinktion wurde das Reaktionsgemisch in einer Schichtdicke von 0,025 mm vermessen.

Tabelle II gibt die ermittelten Ausbeuten der Reaktion mit verschiedenen Katalysatoren wieder.

Tabelle II

| Alkylierung von Diphenylamin mit Nonen | |
|---|---|
| Katalysator | Ausbeute Dinonyl-Diphenylamin (%) |
| Bsp.1 | 27 |
| Bsp.2 | 37 |
| Bsp.3 | 35 |

Das Beispiel belegt die verbesserte Aktivität der erfindungsgemäßen Katalysatoren bei der Diphenylaminalkylierung im Vergleich zum Stand der Technik.

<u>Beispiel 5</u>

<u>Veresterung von Essigsäure und Ethanol</u>

72,0 g Essigsäure und 55,2 g Ethanol wurden in einem 250 ml Dreihalskolben mit Thermometer, Magnetrührer und Rückflusskühler vermischt. Von dem Gemisch wurden etwa 0,5 g entnommen, und der Säuregehalt wurde durch Titration mit 0,1 N Natronlauge gegen Phenolphthalein bestimmt. Das Eduktgemisch wurde mittels eines Ölbades auf 85 °C erhitzt und nach Erreichen der Temperatur mit 1,26 g Katalysator (bezogen auf Trockensubstanz) beaufschlagt.

Mit Zugabe des Katalysators wurde eine Stoppuhr in Gang gesetzt, die zur Ermittlung der Reaktionszeit diente. Im 30-Minuten-Rhythmus wurden nun mit einer Pipette je ca. 0,5 bis 1 g Proben gezogen. Die bei der Probenentnahme mitgerissenen geringen Katalysatormengen beeinflussen die Fortführung der Reaktion und die titrimetrische Säuremessung der Probe nicht wesentlich. Tabelle III zeigt die gemessenen Umsätze nach 30 und 60 Minuten Reaktionszeit.

Tabelle III

| Veresterung von Ethanol und Essigsäure | | |
|---|---|---|
| | Umsatz nach 30 min (%) | Umsatz nach 60 min (%) |
| Bsp. 1 | 10 | 13 |
| Bsp. 2 | 27 | 34 |

Beispiel 6

Dehydratisierung von Cyclohexanol

250 ml Cyclohexanol wurden zusammen mit 5 g gepulvertem Katalysator nach Beispiel 3 in einem Dreihalskolben mit aufgesetzter Vigreux-Kolonne und Destillationsbrücke zum Sieden gebracht. Die bei der Umsetzung entstehenden Produkte Cyclohexen und Wasser wurden kontinuierlich aber die Destillationsbrücke dem Reaktionsraum entzogen. Die kondensierten Mengen an Wasser und Cyclohexen wurden in Abhängigkeit von der Reaktionszeit erfasst. Nachdem etwa 200 ml Cyclohexanol umgesetzt waren, wurden dem Reaktionsgefäß erneut 250 ml Substrat zugesetzt. Dieser Vorgang wurde dreimal wiederholt, ohne dass eine signifikante Abnahme der Reaktionsgeschwindigkeit zu beobachten war. Die Produktbildungsrate für den Katalysator nach Beispiel 3 betrug 1,7 ml/min.

Dieses Beispiel belegt die konstant hohe Reaktivität der Katalysatoren für protonenkatalysierte Reaktionen.

Beispiel 7

Kontinuierliche Reinigung von Xylol

Ein Teil des nach dem Waschen des Produkts nach Beispiel 3 anfallenden Filterkuchens wurde bei 110 °C getrocknet und vorsichtig zerstoßen. Aus den Bruchstücken wurde die Kornfraktion 0,25 mm bis 0,50 mm herausgesiebt. 5 ml dieser Bruchgranulate wurde in einen 10 ml-Rohrreaktor eingebracht, der über eine HPLC-Pumpe kontinuierlich mit technischem Xylol durchströmt wurde. Der Rohrreaktor wurde durch ein thermostatisierbares Ölbad auf 175 °C erhitzt, wobei diese Temperatur während des Versuchs konstant gehalten wurde. Um eine Gasblasenbildung bei dieser Temperatur zu vermeiden, war zwischen dem Reaktor und den ebenfalls installierten Probennahmeventilen ein Rückdruckregler installiert, der den Arbeitsdruck im Reaktor konstant auf 30 bar regelte. Über die HPLC-Pumpe wurde nun ein LHSV-Wert (liquid hourly space velocity: Flüssig-Raumgeschwindigkeit) von 12 h$^{-1}$ eingestellt.

Das verwendete technische Xylol besaß einen Bromindex von 580 mg/100 g, bedingt durch ungesättigte aliphatische Verbindungen. An dem granulierten Katalysator wurden diese ungesättigten Verbindungen vermutlich durch eine lewissäure-katalytisierte Alkylierungsreaktion derart umgesetzt, dass nach der Behandlung des Rohmaterials der Bromindex auf Werte kleiner 2 mg/100 g fiel. Im Laufe der Zeit fand eine Deaktivierung des Katalysators statt, die den Bromindex des behandelten Xylols wieder ansteigen ließ. Nach Erreichen eines Bromindexes von 20 mg/100 g wurde nun die Erschöpfung des Katalysators definiert. Die Menge des während dieser Laufzeit umgesetzten Xylols ist ein direktes Maß für die Katalysatoraktivität. Unter Verwendung des granulierten Katalysators nach Beispiel 3 wurde eine Laufzeit von 18 Tagen erzielt, wobei insgesamt 25,861 Xylols umgesetzt wurden. Im Vergleichstest mit einem kommerziell erhältlichen Katalysator, dem Süd-Chemie Produkt Tonsil® CO 630 G, wurde eine Gesamtlaufzeit von 12 Tagen bei einem Xyloldurchsatz von 17,24 Liter während dieser Zeit erreicht.

Das Beispiel belegt somit eine deutliche Verbesserung der Katalysatoraktivität im vergleich zum Stand der Technik.


**Patentansprüche**

1.  Verfahren zur Herstellung von Katalysatoren durch Säureaktivierung von Schichtsilicaten und Modifizierung mit katalytisch wirksamen Metallionen, **dadurch gekennzeichnet, dass** man die Säureaktivierung in Anwesenheit von zugesetzten katalytisch wirksamen Aluminium- und/oder Cerionen durchführt und die bei der Säureaktivierung entstehende Lösung zusammen mit der die überschüssigen, katalytisch wirksamen Kationen enthaltenden Restlösung abtrennt.

**2.** Verfahren nach Anspruch 1, **dadurch gekennzeichnet, dass** man Schichtsilicate aus der Gruppe der Smektite, Chlorite, Illite, Vermiculite der Serpentin-Kaolin-Gruppe, sowie der Sepiolith-Palygorskit-Gruppe verwendet, insbesondere Montmorillonit, Beidellit und Nontronit.

**3.** Verfahren nach Anspruch 1 oder 2, **dadurch gekennzeichnet, dass** man die Säureaktivierung in Gegenwart der Aluminiumionen enthaltenden Ablauge aus einer früheren Säureaktivierung durchführt.

**4.** Verfahren nach einem der Ansprüche 1 bis 3, **dadurch gekennzeichnet, dass** man die mit den katalytisch wirksamen Metallionen belegten säureaktivierten Schichtsilicate wäscht, trocknet und calciniert.

**5.** Verwendung der nach dem Verfahren nach einem der Ansprüche 1 bis 4 erhaltenen Katalysatoren für protonen- oder lewissäurekatalytisierte Reaktionen, insbesondere zur Umsetzung von höheren Olefinen mit aromatischen Hydroxyverbindungen und Aminen, für Veresterungs- und Dehydratisierungsreaktionen sowie für die Reinigung von Aromaten.

**Claims**

**1.** A method for preparing catalysts by acid activation of layered silicates and modification with catalytically active metal ions, **characterised in that** the acid activation is carried out in the presence of added catalytically active aluminium and/or cerium ions and the solution arising during the acid activation is separated together with the remaining solution containing the excess catalytically active cations.

**2.** A method according to Claim 1, **characterised in that** layered silicates from the group of the smectites, chlorites, illites, vermiculites of the serpentine kaolin group, as well as the sepiolite-palygorskite group, in particular montmorillonite, beidellite and nontronite.

**3.** A method according to Claim 1 or 2, **characterised in that** the acid activation is carried out in the presence of the spent liquor containing aluminium ions and arising from a previous acid activation.

**4.** A method according to any one of Claims 1 to 3, **characterised in that** the acid activated layered silicates coated with the catalytically active metal ions are washed, dried and calcined.

**5.** Use of the catalysts obtained in accordance with the method according to any one of Claims 1 to 4 for protonic acid or Lewis-acid catalysed reactions, in particular for reacting higher olefins with aromatic hydroxy compounds with amines, for esterification and dehydration reactions, and also for the purification of aromatics.

**Revendications**

**1.** Procédé de préparation de catalyseurs par activation acide de phyllosicilates et modification avec des ions métalliques à activité catalytique, **caractérisé en ce que** l'on réalise l'activation acide en présence d'ions ajoutés d'aluminium et/ou de cérium à activité catalytique et que l'on sépare la solution formée lors de l'activation acide conjointement avec la solution résiduelle contenant des cations à activité catalytique en excès.

**2.** Procédé selon la revendication 1, **caractérisé en ce que** l'on utilise des phyllosilicates du groupe des smectites, des chlorites, des illites, des vermiculites du groupe kaolin-serpentine ainsi que du groupe sépiolithe-palygorskite, en particulier la montmorillonite, la beidellite et la nontronite.

**3.** Procédé selon la revendication 1 ou 2, **caractérisé en ce que** l'on réalise l'activation acide en présence de la lessive résiduaire contenant des ions aluminium issue d'une activation acide préalable.

**4.** Procédé selon l'une quelconque des revendications 1 à 3, **caractérisé en ce que** l'on lave, sèche et calcine les phyllosilicates activés à l'acide revêtus des ions métalliques à activité catalytique.

**5.** Utilisation des catalyseurs obtenus selon le procédé selon l'une quelconque des revendications 1 à 4 pour des réactions catalysées par un acide protonique ou un acide de Lewis, en particulier pour la réaction d'oléfines supérieures avec des composés hydroxyle aromatiques et des amines, pour des réactions d'estérification et de déshydratation ainsi que pour la purification de composés aromatiques.